(19) Europäisches Patentamt
European Patent Office
Office européen des brevets

(11) **EP 2 289 418 A1**

(12) **EUROPEAN PATENT APPLICATION**

(43) Date of publication:
**02.03.2011 Bulletin 2011/09**

(51) Int Cl.:
***A61B 8/00*** (2006.01)      ***G06T 7/00*** (2006.01)

(21) Application number: **10171177.8**

(22) Date of filing: **29.07.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(30) Priority: **27.08.2009 KR 20090079732**

(71) Applicant: **MEDISON CO., LTD.**
**Gangwon-do 250-875 (KR)**

(72) Inventor: **Lee, Yun Hee**
**135-851, Seoul (KR)**

(74) Representative: **Schmid, Wolfgang**
**Lorenz & Kollegen**
**Patentanwälte Partnerschaftsgesellschaft**
**Alte Ulmer Strasse 2**
**89522 Heidenheim (DE)**

(54) **Ultrasound system and method for providing a plurality of slice plane images**

(57)    There is disclosed an embodiment for providing a plurality of slice plane images by setting the plurality of slice plane on volume data. An ultrasound data acquisition unit forms ultrasound data of a target object. A processor forms volume data based on the ultrasound data. A user interface receive a first user instruction for setting a reference plane in the volume data, a second user instruction for setting a reference point on the reference plane and a third user instruction for providing information on a plurality of slice planes. The processor sets a plurality of slice planes, each being orthogonal to the reference plane and including the reference point and forms a plurality of slice plane images.

## FIG. 1

EP 2 289 418 A1

**Description**

CROSS-REFERENCE TO RELATED APPLICATIONS

The present application claims priority from Korean Patent Application No. 10-2009-0079732 filed on August 27, 2009, the entire subject matter of which is incorporated herein by reference.

TECHNICAL FIELD

The present invention generally relates to ultrasound systems, and more particularly to an ultrasound system and method for providing a plurality of slice plane images by setting a plurality of slice planes on volume data.

BACKGROUND

An image processing system, which is an apparatus for visualizing an image of a target object through signal processing, has been utilized in various fields. An image processing system for ultrasound diagnosis ("ultrasound system") will be described as an example of the image processing system.

The ultrasound system has become an important and popular diagnostic tool due to its non-invasive and non-destructive nature. Modem high-performance ultrasound imaging diagnostic systems and techniques are commonly used to produce two or three-dimensional images of internal features of patients (target objects). Conventionally, the ultrasound system includes an ultrasound probe, a body, a control panel and a display unit. The ultrasound probe includes a plurality of transducer elements formed by using piezoelectric materials to transmit and receive ultrasound signals. The ultrasound probe forms the ultrasound signals by electrically stimulating the transducer elements to transmit the ultrasound signals into the target objects. Echo signals caused by reflection of the ultrasound signals from the target objects are converted into electrical signals by the transducer elements. The body applies delays to the electrical signals outputted from the probe in consideration of the distances between the transducer elements and focal points to thereby output receive-focused signals. The body forms an ultrasound image indicative of the target objects based on the receive-focused signals. The control panel includes a touch screen, a keyboard, a trackball and a plurality of buttons for allowing a user to input instruction for controlling functions of ultrasound image acquisition, menu control, measurement and annotation.

Conventionally, the ultrasound system displays at least one of reference plane images, e.g., plane images corresponding to sagittal, coronal and axial views, respectively, from a three-dimensional ultrasound image formed based on the ultrasound echo signals. The ultrasound system may allow a user to draw an arbitrary line, e.g., a straight line or a curved line on the reference plane image through manipulation of the control panel.

A function of displaying a plane image in the ultrasound system, which is adopted from a function of displaying a plane image of a computerized tomography (CT) or a magnetic resonance imager (MRI), may merely provide plane images in parallel with the reference plane. Thus, there is a disadvantage in that a variety of plane images cannot be provided.

SUMMARY

An embodiment for forming a plurality of three-dimensional ultrasound images is disclosed herein. In one embodiment, by way of non-limiting example, an ultrasound system for providing a plurality of slice plane images, comprises: an ultrasound data acquisition unit configured to form ultrasound data of a target object; a processor configured to form volume data based on the ultrasound data; and a user interface configured to allow a user to input a first user instruction for setting a reference plane in the volume data, a second user instruction for setting a reference point on the reference plane and a third user instruction for providing information on a plurality of slice planes, wherein the processor is further configured to set a plurality of slice planes, each being orthogonal to the reference plane and including the reference point, and form a plurality of slice plane images.

In another embodiment, a method of providing a plurality of slice plane images, comprises: a) forming ultrasound data of a target object; b) forming volume data based on the ultrasound data; c) receiving a first user instruction for setting a reference plane in the volume data; d) receiving a second user instruction for setting a reference point on the reference plane; e) receiving a third user instruction for providing information on a plurality of slice planes; f) setting a plurality of slice planes, each being orthogonal to the reference plane and including the reference point; and g) forming a plurality of slice plane images corresponding to the plurality of slice planes. In yet another embodiment of the present invention, a computer readable medium comprising instructions that, when executed by a processor performs a plurality of slice plane images providing method of an ultrasound system, cause the processor to perform the above-described steps.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

BRIEF DESCRIPTION OF THE DRAWINGS

FIG. 1 is a block diagram showing an illustrative embodiment of an ultrasound system.

FIG. 2 is a block diagram showing an illustrative embodiment of an ultrasound data acquisition unit in FIG. 1.

FIG. 3 is a block diagram showing an illustrative embodiment of a processor in FIG. 1.

FIG. 4 is a schematic diagram showing an example of volume data and reference planes.

FIG. 5 is a schematic diagram showing an example of volume data, reference planes and slice planes.

DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

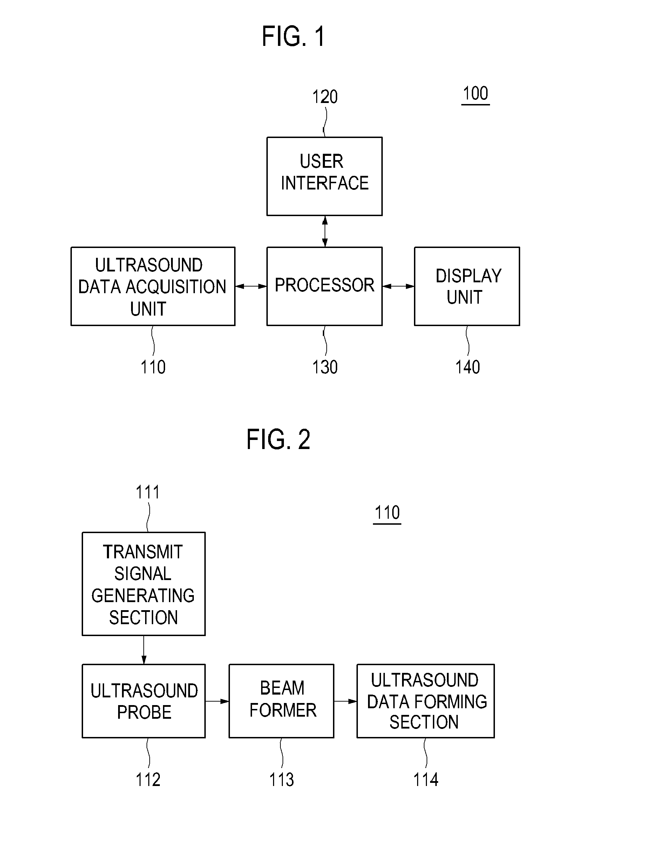

FIG. 1 is a block diagram showing an illustrative embodiment of an ultrasound system. The ultrasound system 100 may include an ultrasound data acquisition unit 110, a user interface 120, a processor 130 and a display unit 140.

The ultrasound data acquisition unit 110 may be configured to transmit ultrasound signals to a target object (not shown) and receive ultrasound echo signals reflected from the target object to thereby acquire ultrasound data.

FIG. 2 is a block diagram showing an illustrative embodiment of an ultrasound data acquisition unit 110. The ultrasound data acquisition unit 110 may include a transmit (Tx) signal generating section 111, an ultrasound probe 112 including a plurality of transducer elements (not shown), a beam former 113 and an ultrasound data forming section 114.

The Tx signal generating section 111 may generate Tx signals according to an image mode set in the ultrasound system 100. The image mode may include a brightness (B) mode, a Doppler (D) mode, a color flow mode, etc. In one exemplary embodiment, the B mode may be set in the ultrasound system 100 to obtain a B mode ultrasound image.

The ultrasound probe 112 may receive the Tx signals from the Tx signal generating section 111 and generate ultrasound signals, which may travel into the target object. The ultrasound probe 112 may further receive ultrasound echo signals reflected from the target object and convert them into electrical receive signals. In such a case, the electrical receive signals may be analog signals. The ultrasound probe 112 may include at least one of a three-dimensional probe, a two-dimensional probe, a one-dimensional probe and the like.

The beam former 113 may convert the electrical receive signals outputted from the ultrasound probe 112 into digital signals. The beam former 113 may further apply delays to the digital signals in consideration of the distances between the transducer elements and focal points to thereby output receive-focused signals.

The ultrasound data forming section 114 may form a plurality of ultrasound data by using the receive-focused signals. In one embodiment, the plurality of ultrasound data may be radio frequency (RF) data or IQ data.

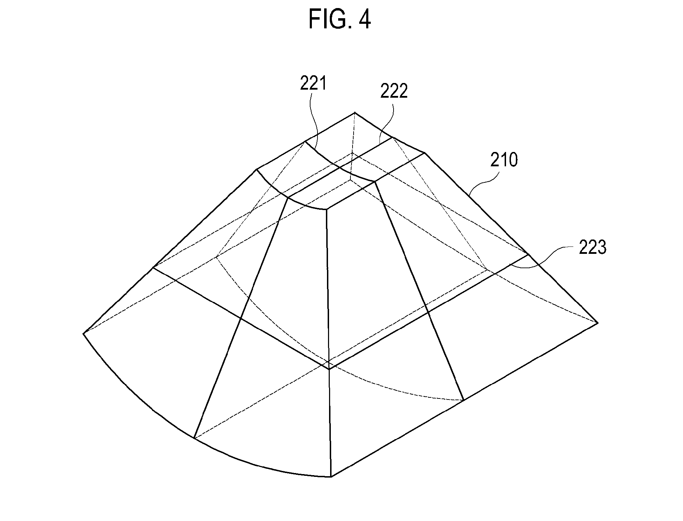

Referring back to FIG. 1, the user interface 120 may include at least one of a control panel (not shown), a mouse (not shown), a keyboard (not shown) and the like. The user interface 120 may be operable to allow a user to input user instructions. The user instructions may include first, second and third user instructions. The first user instruction may be for setting a reference plane in a volume data. The second user instruction may be for setting a reference point on the reference plane. The third user instruction may be for providing information on a plurality of slice planes. The information on a plurality of slice planes may include the number of slice planes or an angle between the slice planes. In one embodiment, by way of non-limiting examples, the reference planes may include an A plane 221, a B plane 222 and a C plane 223, being orthogonal each other in a volume data 210, as illustrated in FIG. 4. The volume data 210 may be formed by arraying a plurality of frames based on the ultrasound data and interpolating between the plurality of frames.

The processor 130 may be configured to form volume data based on ultrasound data provided from the ultrasound data acquisition unit 110. The processor 130 may be further configured to form a plurality of slice plane images based on the user instructions provided from the user interface 120.

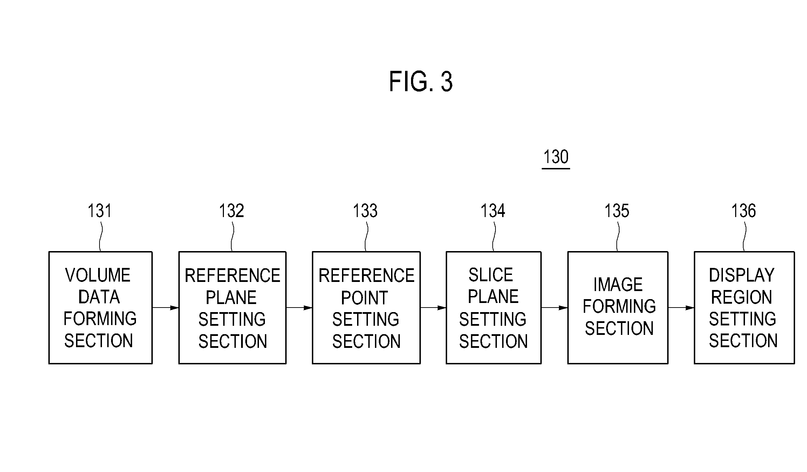

FIG. 3 is a block diagram showing an illustrative embodiment of the processor 130 in FIG. 1. The processor 130 may include a volume data forming section 131, a reference plane setting section 132, a reference point setting section 133, a slice plane setting section 134, an image forming section 135 and a display region setting section 136.

The volume data forming section 131 may be configured to form the volume data 210 based on the ultrasound data provided from the ultrasound data acquisition unit 110. The volume data may include a plurality of frames and voxels. Each voxel may have brightness intensity.

The reference plane setting section 132 may set the reference plane in the volume data according to the first user instruction provided from the user interface 120. In one embodiment, by way of non-limiting examples, the A plane 221 may be set as the reference plane.

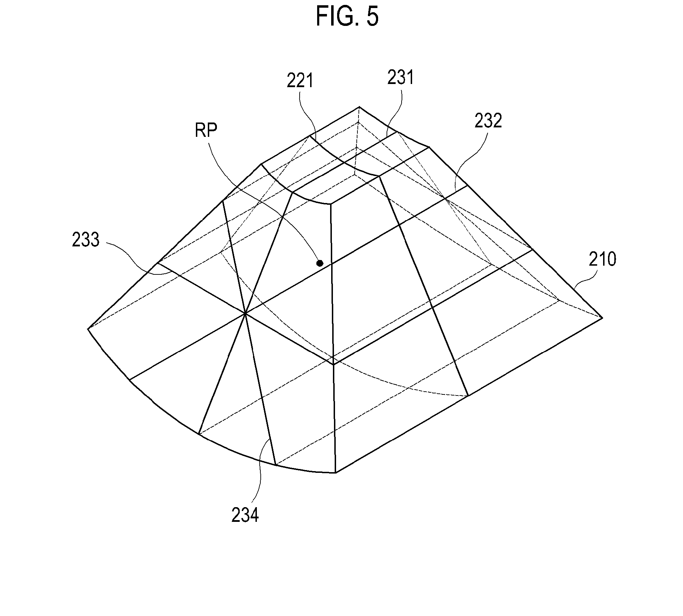

FIG. 5 is a schematic diagram showing an example of volume data, reference planes and reference point. The reference point setting section 133 may set a reference point RP on the reference plane 221 according to the second user instruction provided from the user interface 120.

The slice plane setting section 134 may set a plurality of slice planes 231-234 according to the third instruction provided from the user interface 120. Each of the slice planes 231-234 may be set to be perpendicular to the reference plane 221 and include the reference point RP.

If the third user instruction includes the angle between neighboring slice planes, then the slice plane setting section 134 calculates the number of slice planes according to the following equation (1).

$$n = (360° / \theta) / 2 \qquad (1)$$

wherein n denotes the number of slice planes and θ denotes the angle between the neighboring slice planes. The slice plane setting section 134 may set the slice planes 231-234 in the volume data 210 according to the calculated number of slice planes, as illustrated in FIG. 5.

If the third user instruction includes the number of slice planes, then the slice plane setting section 134 sets the slice planes 231-234 in the volume data 210 according to the number of slice planes through the reference point RP.

Although it is described above that the slice planes 231-234 are set to have the same angle between neighboring slice planes, the angle in-between is not limited thereto. In another embodiment, the slice planes may be set differently from each other.

The image forming section 135 may form a plurality of slice plane images corresponding to the plurality of slice planes 231-234 by using the volume data. Further, the image forming section 135 may form a reference plane image corresponding to the reference plane 221 by using the volume data.

The display region setting section 136 may set display regions of the display unit 140 to display the slice plane images based thereon. The display region setting section 136 may set the display regions as many as the number of slice plane images. Furthermore, the display region setting section 136 may set the display regions to display the slice plane images together with the reference plane image.

Referring back to FIG. 1, the display unit 140 may display the slice plane images at the relative display regions. Further, the display unit 140 may display the reference plane images at the display regions. The display unit 140 may include a cathode ray tube (CRT) display, a liquid crystal display (LCD), organic light emitting diodes (OLED) display and the like.

As described above according to one embodiment, the plurality of slice plane images may be set such that the angle between the neighboring slice plane images is identically or differently. Thus, since the slice plane images can be acquired at various directions, it may be easy and useful to compare and analyze the slice plane images.

In one embodiment, instructions for performing the above method of providing the slice plane images may be recorded in a computer readable medium using computer-readable instructions. The computer readable medium may include any type of record media, which can be read by a computer system. The computer readable medium may include read only memory (ROM), random access memory (RAM), CD-ROM, magnetic tape, floppy disk, optical-data recording apparatus and the like. The computer readable medium comprises instructions that, when executed by a processor performs a plurality of slice plane images providing method of an ultrasound system, cause the processor to perform the following steps: a) forming ultrasound data of a target object; b) forming volume data based on the ultrasound data; c) receiving a first user instruction for setting a reference plane in the volume data; d) receiving a second user instruction for setting a reference point on the reference plane; e) receiving a third user instruction for providing information on a plurality of slice planes; f) setting a plurality of slice planes, each being orthogonal to the reference plane and including the reference point; and g) forming a plurality of slice plane images corresponding to the plurality of slice planes.

Any reference in this specification to "one embodiment," "an embodiment," "example embodiment," "illustrative embodiment," etc. means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to affect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

**Claims**

1.  An ultrasound system, comprising:

an ultrasound data acquisition unit configured to form ultrasound data of a target object;
a processor configured to form volume data based on the ultrasound data; and

a user interface configured to allow a user to input a first user instruction for setting a reference plane in the volume data, a second user instruction for setting a reference point on the reference plane and a third user instruction for providing information on a plurality of slice planes,

wherein the processor is further configured to set a plurality of slice planes,

each being orthogonal to the reference plane and including the reference point,

and form a plurality of slice plane images.

**2.** The ultrasound system of Claim 1, further comprises:

a display unit configured to display the slice plane images.

**3.** The ultrasound system of Claim 2, wherein the processor comprises:

a volume data forming section configured to form the volume data based on the ultrasound data;

a reference plane setting section configured to set the reference plane in the volume data according to the first user instruction;

a reference point setting section configured to set the reference point in the reference plane according to the second user instruction;

a slice plane setting section configured to set a plurality of slice planes according to the third instruction;

an image forming section configured to form a plurality of slice plane images according to the plurality of slice planes set in the volume data; and

a display region setting section configured to set display regions of display unit as many as the number of slice plane images.

**4.** The ultrasound system of Claim 3, wherein the image forming section forms the reference plane images according to the reference plane set in the volume data and the display region setting section sets the display regions to display the slice plane images together with the reference plane image.

**5.** A method of providing slice plane images in an ultrasound system, comprising:

a) forming ultrasound data of a target object;
b) forming volume data based on the ultrasound data;
c) receiving a first user instruction for setting a reference plane in the volume data;
d) receiving a second user instruction for setting a reference point on the reference plane;

e) receiving a third user instruction for providing information on a plurality of slice planes;
f) setting a plurality of slice planes, each being orthogonal to the reference plane and including the reference point; and
g) forming a plurality of slice plane images corresponding to the plurality of slice planes.

**6.** The method of Claim 5, further comprising:

h) setting display regions of a display unit as many as the number of slice plane images.

**7.** The method of Claim 6, wherein the step g) comprises forming the reference plane images according to the reference plane set in the volume data and the step h) comprises setting the display regions to display the slice plane images together with the relative reference plane image.

**8.** A computer readable medium comprising instructions that, when executed by a processor performs a plurality of slice plane images providing method of an ultrasound system, cause the processor to perform steps comprising:

a) forming ultrasound data of a target object;
b) forming volume data based on the ultrasound data;
c) receiving a first user instruction for setting a reference plane in the volume data;
d) receiving a second user instruction for setting a reference point on the reference plane;
e) receiving a third user instruction for providing information on a plurality of slice planes;
f) setting a plurality of slice planes, each being orthogonal to the reference plane and including the reference point; and
g) forming a plurality of slice plane images corresponding to the plurality of slice planes.

**9.** The computer readable medium of Claim 8, further comprising:

h) setting display regions of a display unit as many as the number of slice plane images.

**10.** The computer readable medium of Claim 9, wherein the step g) comprises forming the reference plane images according to the reference planes set in the volume data and the step h) comprises setting the display regions to display the slice plane images and the relative reference plane image together.

# FIG. 1

120

100

USER
INTERFACE

ULTRASOUND
DATA ACQUISITION
UNIT

PROCESSOR

DISPLAY
UNIT

110

130

140

# FIG. 2

111

110

TRANSMIT
SIGNAL
GENERATING
SECTION

ULTRASOUND
PROBE

BEAM
FORMER

ULTRASOUND
DATA FORMING
SECTION

112

113

114

## FIG. 3

130

| 131 | 132 | 133 | 134 | 135 | 136 |
|---|---|---|---|---|---|
| VOLUME DATA FORMING SECTION | REFERENCE PLANE SETTING SECTION | REFERENCE POINT SETTING SECTION | SLICE PLANE SETTING SECTION | IMAGE FORMING SECTION | DISPLAY REGION SETTING SECTION |

FIG. 4

FIG. 5

Europäisches
Patentamt

European
Patent Office

Office européen
des brevets

## EUROPEAN SEARCH REPORT

Application Number

EP 10 17 1177

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2005/031649 A1 (SIEMENS MEDICAL SOLUTIONS [US]; CATHIER PASCAL [US]) 7 April 2005 (2005-04-07) * page 5, paragraph 3 * * page 8 - page 9 * * figures 2-5 * ----- | 1-10 | INV. A61B8/00 G06T7/00 |
| Y | SIMON M ET AL: "PADDLE-WHEEL CT DISPLAY OF PULMONARY ARTERIES AND OTHER LUNG STRUCTURES: A NEW IMAGING APPROACH", AMERICAN JOURNAL OF ROENTGENOLOGY, AMERICAN ROENTGEN RAY SOCIETY, US, vol. 177, no. 1, 1 July 2001 (2001-07-01), pages 195-199, XP009057509, ISSN: 0361-803X * the whole document * ----- | 1-10 | |
| Y | US 2005/261567 A1 (HARDER MARTIN [DE] ET AL) 24 November 2005 (2005-11-24) * paragraph [0014] * * paragraph [0023] - paragraph [0028] * * figure 2 * ----- | 1-10 | |
| Y | US 7 376 254 B2 (BARTH KARL [DE]) 20 May 2008 (2008-05-20) * column 8, line 44 - column 9, line 15 * * column 10, line 49 - line 53 * * figures 2-5 * ----- -/-- | 1-10 | TECHNICAL FIELDS SEARCHED (IPC) A61B G06T G01S |

The present search report has been drawn up for all claims

| Place of search | Date of completion of the search | Examiner |
|---|---|---|
| The Hague | 3 December 2010 | Dydenko, Igor |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

Europäisches
Patentamt

European
Patent Office

Office européen
des brevets

**EUROPEAN SEARCH REPORT**

Application Number

EP 10 17 1177

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SIMON M ET AL: "PADDLE-WHEEL MULTISLICE HELICAL CT DISPLAY OF PULMONARY VESSELS AND OTHER LUNG STRUCTURES", RADIOLOGIC CLINICS OF NORTH AMERICA, SAUNDERS, PHILADELPHIA, PA, US, vol. 41, no. 3, 1 May 2003 (2003-05-01), pages 617-626, XP009057950, ISSN: 0033-8389, DOI: DOI:10.1016/S0033-8389(03)00024-1 * the whole document * | 1-10 | |
| A | EP 1 757 229 A1 (MATSUSHITA ELECTRIC IND CO LTD [JP] PANASONIC CORP [JP]) 28 February 2007 (2007-02-28) * paragraph [0037] * * figure 8 * | 1-10 | |
| A | EP 1 780 671 A1 (MEDISON CO LTD [KR]) 2 May 2007 (2007-05-02) * paragraphs [0017], [0 23] - [0024] * * figure 4 * | 1-10 | |

TECHNICAL FIELDS
SEARCHED    (IPC)

The present search report has been drawn up for all claims

| Place of search | Date of completion of the search | Examiner |
|---|---|---|
| The Hague | 3 December 2010 | Dydenko, Igor |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

      .....................................................................................
& : member of the same patent family, corresponding
     document

EPO FORM 1503 03.82 (P04C01)

## ANNEX TO THE EUROPEAN SEARCH REPORT
## ON EUROPEAN PATENT APPLICATION NO.

EP 10 17 1177

This annex lists the patent family members relating to the patent documents cited in the above-mentioned European search report.
The members are as contained in the European Patent Office EDP file on
The European Patent Office is in no way liable for these particulars which are merely given for the purpose of information.

03-12-2010

| Patent document cited in search report | | Publication date | Patent family member(s) | | Publication date |
|---|---|---|---|---|---|
| WO 2005031649 | A1 | 07-04-2005 | CN 1853197 A | | 25-10-2006 |
| | | | DE 112004001691 T5 | | 20-07-2006 |
| | | | JP 4463817 B2 | | 19-05-2010 |
| | | | JP 2007505711 T | | 15-03-2007 |
| US 2005261567 | A1 | 24-11-2005 | CN 1695550 A | | 16-11-2005 |
| | | | DE 102004023849 A1 | | 08-12-2005 |
| US 7376254 | B2 | 20-05-2008 | DE 10254907 A1 | | 17-06-2004 |
| | | | US 2004160440 A1 | | 19-08-2004 |
| EP 1757229 | A1 | 28-02-2007 | CN 101014290 A | | 08-08-2007 |
| | | | WO 2005110237 A1 | | 24-11-2005 |
| | | | US 2007249937 A1 | | 25-10-2007 |
| EP 1780671 | A1 | 02-05-2007 | JP 2007125393 A | | 24-05-2007 |
| | | | KR 20070047007 A | | 04-05-2007 |
| | | | US 2007110291 A1 | | 17-05-2007 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020090079732